# EUROPEAN PATENT APPLICATION

(11) **EP 4 450 192 A1**
(43) Date of publication of application: **23.10.2024**
(21) Application number: 24169869.5
(22) Date of filing: 12.04.2024
(51) Int. Cl.: B22F 10/47, B22F 5/00, B22F 10/66, B22F 5/10, B33Y 10/00, B33Y 80/00, B28B 1/00, B29C 64/153, B29C 64/40, A61F 2/00, B22F 10/28

(54) **IMPLANT SUPPORT STRUCTURE AND METHOD OF FABRICATION OF IMPLANT USING THE SAME**

(30) Priority: 12.04.2023 US 202363458748 P
(71) Applicant: Howmedica Osteonics Corp., Mahwah, NJ 07430 (US)
(72) Inventor: CROUS, Heinrich George, CARRIGTWOHILL, T45 RF20 (IE)
(74) Representative: Regimbeau

(57) **Abstract**

An in-process build assembly (100) includes an in-process build structure (110), a support structure (120), and semi-sintered particles. The support structure supports the in-process build structure. The support structure surrounds a cross-section of the in-process build structure without surrounding an entirety of the in-process build structure. The semi-sintered particles are located between the in-process build structure and the support structure such that there is no direct attachment of the in-process build structure to the support structure. The in-process build assembly is fabricated by an additive manufacturing process. Either one of or both the in-process build structure and the support structure are moved after the additive manufacturing process such that the cross-section of the in-process build structure is outside the support structure.

## Description

### CROSS-REFERENCE TO RELATED APPLICATION

The present application claims the benefit of the filing date of U.S. Provisional Application No. 63/458,748, filed April 12, 2023, the entirety of the disclosure of which is hereby incorporated herein by reference.

### FIELD

The present invention generally relates to additive manufacturing, and in particular relates to an in-process support for use in fabricating an object, especially an elongated object such as a femoral hip stem, without directly contacting the object and a process for making the support and the object via an additive manufacturing process.

### BACKGROUND

Additive manufacturing (AM) processes involve the buildup of one or more materials to make or form an object. These processes encompass various manufacturing and prototyping techniques known under a variety of names, including solid freeform fabrication, three-dimensional (3D) printing, and rapid manufacturing/prototyping. AM techniques are capable of fabricating components having complex geometries from a wide variety of materials. Objects formed by AM processes generally are fabricated using a computer-aided design (CAD) model. Certain types of AM processes use a high energy beam, for example, an electron beam or electromagnetic radiation such as a laser beam, to sinter or melt a fine powder material, creating a solid 3D object in which particles of the powder material are bonded together. Applications include direct manufacturing of complex workpieces, patterns for investment casting, metal molds for injection molding and die casting, and molds and cores for sand casting.

Selective laser sintering (SLS), direct metal laser sintering (DMLS), selective laser melting (SLM), electron beam melting (EBM), are all common AM processes that involve successively depositing and heating layers of powder material to fuse the powder material, e.g., metallic powder, and thereby produce 3D objects as described, for example, in U.S. Patent Nos. 11,510,783 and 10,716,673, the disclosures of which are hereby incorporated by reference herein. After heat is applied to the powder to induce sintering or melting, the layers of powder and indeed the object once initially formed retains the applied heat for a period of time. Portions of the object that remain heated are often in a weaker state than they are once the heat has dissipated. Certain objects may be formed by applying the entirety of sequential layers directly on top of previous layers or a build plate, in the case of a first layer, in which the respective build plate or previous layers fully support their immediately following layers.

However, other objects desirably may be formed with a base layer or layers supported by the build plate and a free hanging portion of the object extending laterally from the base layer without support from the build plate or previous layers. As such, free hanging portions are more susceptible to deformation after heat is applied to form those portions. In such circumstances, powder bed fusion supports attached to objects are often needed during fabrication of the objects to prevent such deformation. Such supports require additional post-processing methods to have them removed, which can be costly and involve time-consuming additional steps. Support remnants are sometimes left on critical-to-quality surfaces such that longer or additional cycles of post-processing, e.g., sandblasting, are needed to remove powder trapped between supports, especially at contoured surfaces. The use of these breakaway-type supports also leads to the need for costly and time-consuming powder recycling operations. As an alternative, contactless heat sinks, which do also necessitate undesirable recycling operations, have been used in place of attached supports to draw heat away from free hanging portions, as described, for example, in U.S. Patent Appl. Publ. No. 2022/0410272 A1, the disclosure of which is hereby incorporated by reference herein. However, maintaining the stability of many intended objects during an AM process requires that the objects be attached, directly or via an attached support, to an associated build plate during the process. In such instances, post-processing is required to separate the AM fabricated objects from their associated build plate and to address any remnants left on such objects upon their removal from the build plates.

Hence, further enhancements are needed to reduce or eliminate the use of these supports and heat sinks and thereby improve part quality, improve process efficiency through reductions in post-processing operations, and reduce processing and material costs.

### BRIEF SUMMARY

In accordance with an aspect, a first portion of a support may be fabricated, e.g., via an additive manufacturing process, onto a substrate. The substrate may be a build plate or another preformed structure. A second portion of the support may be fabricated along with a first portion of an object intended to be formed, e.g., as a set of successive fused powder layers via an additive manufacturing process, such that a perimeter of the first portion of the object fabricated with the second portion of the support is surrounded by the second portion of the support while a second portion of the object extends beyond the support and is directly supported solely by the first portion of the object. The object preferably lacks any direct attachment to the substrate during the fabrication process. In some arrangements, the support may be fixed to the substrate upon the fabrication of the support. In some such arrangements, central axes of the second portion of the support and the first portion of the object surrounded by the second portion of the support may extend at an oblique angle to the substrate. In some arrangements, a powder that is not fully sintered may attach the object to the support such that the object and the support lack any direct attachment.

In accordance with another aspect of the disclosure, a build structure may be fabricated by a process. In the process, a support structure supported by a build plate may be fabricated, via an additive manufacturing process. In the process, an in-process build structure may be fabricated, via the additive manufacturing process, such that the support structure surrounds a cross-section of the in-process build structure. Preferably, the in-process build structure may be fabricated such that an entirety of the in-process build structure is not surrounded by the support structure. In the process, either one of or both the in-process build structure and the support structure may be moved such that the cross-section of the in-process build structure is outside the support structure. In some arrangements, the in-process build structure may be removed or at least separated from the support structure.

In some arrangements according to this aspect, the in-process build structure may be attached to the support structure with semi-sintered particles. In some such arrangements, immediately upon completion of the fabrication of the support structure and the in-process build structure, the in-process build structure may be attached to and supported by the support structure via the semi-sintered particles without the in-process build structure being directly attached to the support structure. In some such arrangements, the support structure may be fixed to a build plate, e.g., of an additive manufacturing machine, without the in-process build structure being directly attached to the build plate.

In some arrangements according to this aspect, fabrication of the in-process build structure and the support structure may form the in-process build structure and the support structure such that a first portion of the in-process build structure that includes the cross-section of the in-process build structure may be held within the support structure.

In some arrangements according to this aspect, the fabrication of the support structure and the in-process build structure may include applying a high energy beam onto a powder layer to form the first portion of the in-process build structure, a first portion of the support structure, and semi-sintered particles between the in-process build structure and the support structure.

In any of the arrangements according to this aspect, a first surface of the in-process build structure may be altered when the in-process build structure is outside the support structure. In some such arrangements, the alteration of the first surface may include lowering a surface roughness of a first region of the first surface of the in-process build structure. In some such arrangements, the first surface of the first region may be polished.

In some arrangements according to this aspect, the alteration of the first surface may include sandblasting the support structure attached to the in-process support structure.

In any of the arrangements according to this aspect, the in-process build structure may be either one of or both twisted and pulled relative to the support structure such that the in-process build structure may be detached from the support structure. In some such arrangements, the support structure may be blasted during a removal of powder during an additive manufacturing process. In this manner, semi-sintered particles may be loosened to such an extent that the in-process build structure and the support structure may be separated from each other by the twisting and pulling of the in-process build structure relative to the support structure.

In some arrangements according to this aspect, the in-process build structure may be fabricated in the form of a femoral hip stem. In some such arrangements, the in-process build structure may be fabricated such that the support structure surrounds a portion of the in-process build structure in the form of a trunnion head of a femoral hip stem.

In some arrangements according to this aspect, either one of or both the support structure and the in-process build structure may be made of any one or any combination of a metal material, a polymeric or other plastic material, or a ceramic material. In some such arrangements, the support structure may be made of a first metal and the in-process build structure may be made of a second metal different from the first metal. In some arrangements, the metal material may be a cobalt chrome alloy, a titanium or alloy, a stainless steel, niobium or tantalum, or any combination thereof. In some arrangements, one of the support structure or the in-process build structure may be made of a metal while the other one of the support structure or the in-process build structure is made of a non-metallic material, e.g., a ceramic material or a plastic material.

In accordance with another aspect of the disclosure, a build structure may be fabricated by a process. In this process, a first set of layers of powder may be sintered or melted, successively, to fabricate a first portion or portions of a support structure that may be supported by a build plate. In the process, a high energy beam may be applied to a second set of layers of powder, successively, (i) to sinter or melt a first portion or portions of the second set of layers of powder to fabricate a second portion or portions of the support structure supported by the first portion or portions of the support structure, (ii) to heat but no more than semi-sinter a second portion or portions of the second set of layers of powder, and (iii) to sinter or melt a third portion or portions of the second set of layers of powder to fabricate a first portion or portions of an in-process build structure such that the first portion or portions of the second set of layers of powder are spaced from the third portion or portions of the second set of layers of powder by the second portion or portions of the second set of layers of powder. In this process, a third set of layers of powder may be sintered or melted, successively, to fabricate a second portion or portions of the in-process build structure that may be supported by the first portion or portions of the in-process build structure. In this process, the second portion or portions of the second set of layers powder may be between the first portion or portions of the second set of layers of powder and the third portion or portions of the second set of layers of powder.

In some arrangements according to this aspect, the high energy beam may be applied to second layers of powder such that the support structure surrounds a cross-section of the in-process build structure. In some such arrangements, after the fabrication of the second portion or portions of the in-process build structure, the support structure may not surround an entirety of the in-process build structure. In some such arrangements, after the fabrication of the support structure and before completion of the fabrication of the second portion or portions of the in-process build structure, the support structure may not surround an entirety of the in-process build structure.

In any of the arrangements according to this aspect, the high energy beam may be applied to the second set of layers of powder such that the heated but no more than semi-sintered second portion or portions of the second set of layers of powder are surrounded by the second portion or portions of the support structure and the first portion or portions of the in-process build structure are surrounded by the heated but no more than semi-sintered second portion or portions of the second set of layers of powder.

In accordance with another aspect of the disclosure, an in-process build assembly may include an in-process build structure, a support structure, and semi-sintered particles. The support structure may support the in-process build structure and may surround a cross-section of the in-process build structure without surrounding an entirety of the in-process build structure. The semi-sintered particles may be located between the in-process build structure and the support structure such that there may be no direct attachment of the in-process build structure to the support structure.

In any of the arrangements according to this aspect, the support structure may include a receptacle and a buttress extending from the receptacle. In some such arrangements, the receptacle may surround the cross-section of the in-process build structure.

In some arrangements of this aspect having a receptacle, the receptacle may include a plurality of holes. In some arrangements, the plurality of holes may be windows, which may be evenly spaced apart around a perimeter of the receptacle. In some arrangements, the plurality of holes may be perforations located around a perimeter of the receptacle. In some arrangements, the receptacle may be at least partially formed of intersecting struts defining the plurality of holes. In some such arrangements, the intersecting struts may define unit cells.

In any of the arrangements of this aspect having a receptacle, the semi-sintered particles may be located in and supported by the receptacle.

In any of the arrangements of this aspect having a receptacle, the in-process build structure may include a main portion and a protuberance extending from the main portion. In some such arrangements, the cross-section of the in-process build structure may be a cross-section of the protuberance. In some such arrangements, the receptacle may surround the cross-section of the protuberance. In some such arrangements, the receptacle may not surround an entirety of the main portion or may not surround any part of the main portion.

In any of the arrangements of this aspect having a receptacle, the in-process build structure may be attached to the support structure via the semi-sintered particles such that the in-process build structure may be detachable from the support structure merely by either one of or both twisting and pulling the in-process build structure relative to the support structure.

In accordance with another aspect of the disclosure, an in-process build system may include the in-process build assembly in accordance with any of the arrangements of the foregoing aspect having a receptacle. In some such arrangements, the in-process build system may include a build plate attached to the receptacle of the support structure such that the build plate may fully support the in-process build assembly.

In some arrangements according to this aspect, a first portion of the in-process build structure may overlie the build plate and not the support structure. In some such arrangements, the first portion of the in-process build structure may be greater than or equal to 20% by mass of the support structure.

In any of the arrangements according to this aspect, the in-process build structure may include a shaft and the receptacle may include a central cavity into which the shaft of the in-process build structure extends. In some such arrangements, the central cavity of the receptacle may define a central axis that may extend through the shaft of the in-process build structure. In some such arrangements, the central axis may form an angle greater than twenty degrees (20°) with a surface of the build plate supporting the support structure. In some such arrangements, the central axis may form an angle less than ninety degrees (90°) with the surface of the build plate supporting the support structure. In some such arrangements, the central axis may form an angle less than seventy degrees (70°) with the surface of the build plate supporting the support structure.

### BRIEF DESCRIPTION OF THE DRAWINGS

A more complete appreciation of the subject matter of the present invention and various advantages thereof may be realized by reference to the following detailed description, in which reference is made to the following accompanying drawings, in which:
FIG. 1 is a perspective view of an in-process build assembly with a portion of an in-process build structure extending within a support structure in accordance with an embodiment;
FIG. 2 is a cross-sectional view of the in-process build assembly of FIG. 1;
FIG. 3 is an expanded elevation view of the in-process build assembly of FIG. 1;
FIG. 4 is a perspective view of the support structure of the in-process build assembly of FIG. 1;
FIG. 5 is a schematic plan view of the in-process build assembly of FIG. 1 showing an angle of a central axis defined by the support structure and the in-process build structure extending within the support structure relative to a substrate supporting the support structure, in accordance with another embodiment; and
FIGS. 6 and 7 are process flow diagrams of processes for fabricating a build structure in accordance with still other embodiments.

### DETAILED DESCRIPTION

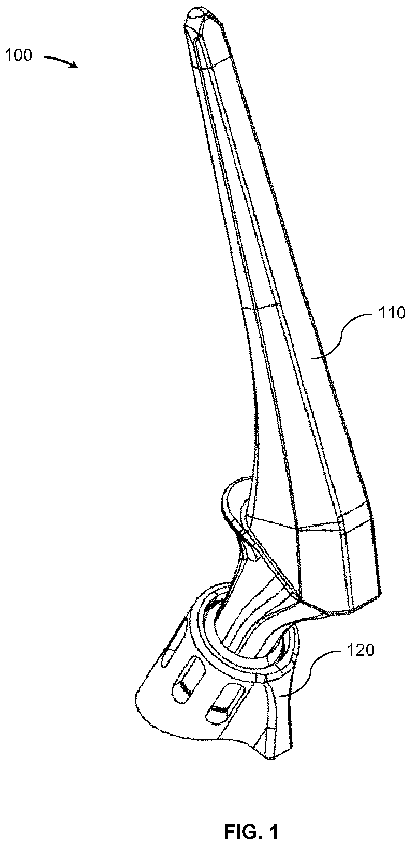

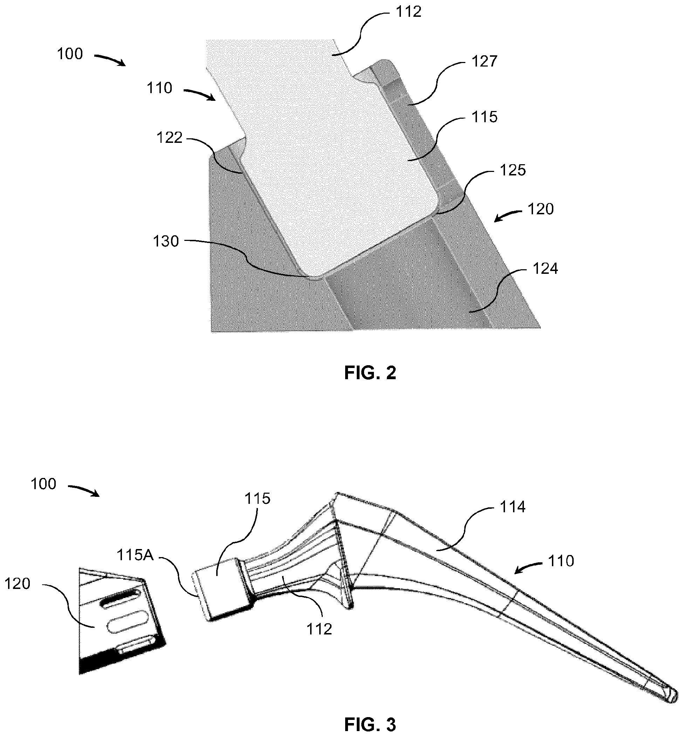

Referring now to the drawings, as shown in FIGS. 1 and 2, in-process build assembly 100 generally includes in-process build structure 110, which is an object in the process of being formed into an intended configuration, and support structure 120 configured for maintaining an end portion of the in-process build structure and thereby supporting the in-process build structure. Although not shown in the figures, during fabrication of in-process build assembly 100, powder particles extend in gap 130 between in-process build structure 110 and support structure 120 such that the in-process build structure is not directly attached to the support structure. In this manner, and with reference to FIG. 3, in-process build structure 110 is separable from a remainder of in-process build assembly 100 without leaving any remnants of the remainder of the in-process build assembly, including from support structure 120, permanently fixed to the in-process build structure upon the separation.

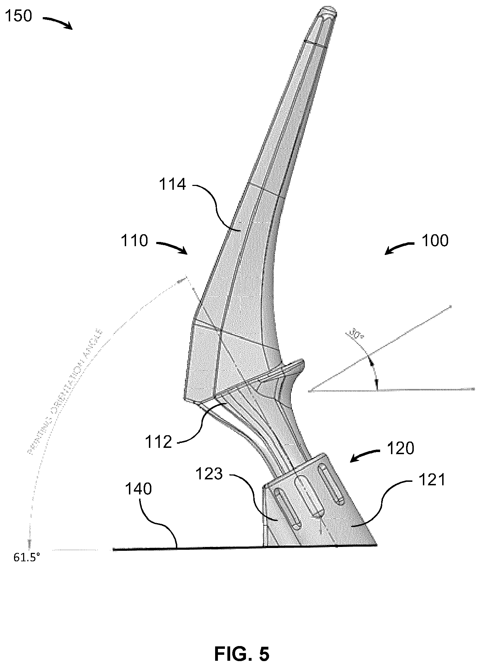

Still referring to FIG. 3 and again referring to FIG. 2, in-process build structure 110 may be in the form of an elongated object, e.g., a femoral hip stem that generally includes neck 112, stem 114, and trunnion 115. As in the example shown, in such arrangements, stem 114 and trunnion 115 of in-process build structure 110 are attached to opposing ends of neck 112 of the build structure. As further shown, stem 114 tapers away from and extends at an oblique angle to neck 112. In this manner, a longitudinal axis of stem 114 extends at an oblique angle, in this case an obtuse angle, relative to a trunnion central axis passing through trunnion 115 of in-process build structure 110 such that the build structure is unbalanced in mass and thereby weight about the trunnion central axis. Due to this unbalanced nature, solid freeform fabrication, e.g., fabrication of a set of successive fused powder layers via additive manufacturing, of in-process build structure 110 in the form of a femoral hip stem normally requires one or more supports attached along a length of the in-process build structure and extending to a substrate (e.g., build plate 140 shown in FIG. 5) supporting the build structure when formed layers of the build structure are stacked in a vertical direction parallel or generally parallel to the trunnion central axis.

With reference to FIGS. 2, 4, and 5, support structure 120, when attached to a substrate such as build plate 140, in conjunction with powder particles in gap 130 operate together as in-process build system 150 to maintain trunnion 115 within support structure 120 during a solid freeform fabrication process, e.g., an additive manufacturing process as discussed further herein. Support structure 120 includes receptacle 121 and buttress 123 protruding from the receptacle. Receptacle 121 includes upper central cavity 122 and lower central bore 124 offset from the upper central cavity by inner step 125 extending between an end of the upper central cavity and an end of the lower central bore. A portion of in-process build structure 110 lies within, and indeed may be fabricated as discussed further herein, within upper central cavity 122. An outer diameter of lower central bore 124, i.e., an inner diameter of inner step 125, may be configured such that in-process build structure 110 overlies the inner step and is unable to be received within the lower central bore, as best shown in FIG. 2.

Still referring to FIG. 2, in some arrangements, the powder particles extending in gap 130 between in-process build structure 110 and support structure 120 are semi-sintered particles attached to both the build and support structures such that the in-process build structure is detachable from, i.e., removable from indirect attachment with, the support structure merely by either one of or both twisting and pulling the in-process build structure relative to the powder particles and thereby relative to the support structure. The semi-sintered particles are heated such that any remaining remnants of the semi-sintered particles left on in-process build structure 110 upon separation of the in-process build structure from the remainder of in-process build assembly (i.e., from the remaining combination of support structure 120 and the semi-sintered particles) is removable without any fracture or other deformity being imparted on the in-process build structure.

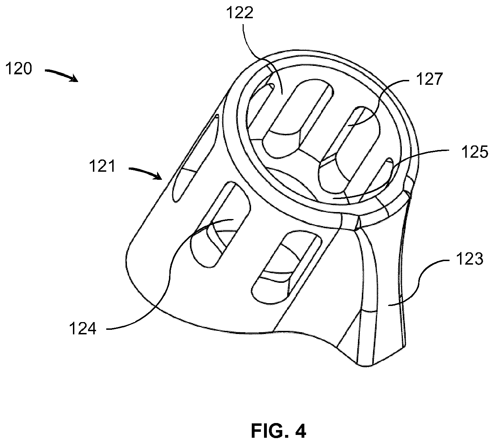

With reference again to FIGS. 2 and 4, receptacle 121 further includes windows 127 extending around a circumference of, and through a thickness of, the receptacle. As in the example shown, windows 127 may be evenly spaced apart except where the windows would otherwise be aligned with buttress 123. In the example shown, receptacle 121 includes a total of seven (7) windows 127, although greater or fewer numbers of windows may be utilized. Windows 127 allow for the removal of loose or otherwise unbound powder from receptacle 121 through the windows, whether or not the receptacle remains attached to build plate 140. When support structure 120 is removed from build plate 140 to which it had been attached, lower central bore 124 is sufficiently large such that loose and otherwise unbound powder passes through the central bore for ease of separation of the powder from the receptacle and therefore ease of recyclability of the powder. As needed, a brush may be used to remove unbound powder from receptacle 121, whether or not the receptacle remains attached to build plate 140.

As best shown in FIG. 5, during a fabrication process, both receptacle 121 and buttress 123 extend from and are supported by build plate 140 such that a receptacle central axis passing through the receptacle extends at an oblique angle to build plate 140. In the example shown, receptacle central axis extends through a plane defined by a surface of build plate 140 on which in-process build structure is fabricated to form an angle of thirty degrees (61.5°) with the defined plane, although greater or lesser angles are possible, e.g., angles in a range from at least fifteen degrees (15°) to less than ninety degrees (90°). At that angle and given the free hanging nature of the thick region of femoral hip stem 110 where neck 112 is joined to stem 114, the configuration of buttress 123 shown, in which an upper end of the buttress intersects with an upper end receptacle 121 and in which a side of the buttress extends downward to build plate 140 from the upper end of the receptacle, is sufficient to support a completely fabricated in-process build structure 110 in the form of a femoral hip stem as shown. In this configuration, at least twenty percent (20%) of the mass and thereby weight of in-process build structure 110, which percentage may vary depending on the angle at which the in-process build structure is oriented during fabrication, e.g., during an AM process, may overlie build plate 140 without overlying any portion of support structure 120 when viewed in a downward direction orthogonal to build plate 140.

The orientation of receptacle central axis is selected via a user, or determined automatically via a computer processor, during CAD modeling to minimize the number of downward facing surfaces (i.e., surfaces facing build plate 140) on in-process build structure 110 that form an angle of thirty degrees (30°) or less with the surface of build plate 140. Receptacle 121 can be configured with a receptacle central axis at a relatively lower angle, e.g., twenty degrees (20°) or forty degrees (40°), relative to build plate 140 by enlarging buttress 123 in a direction away from the receptacle (e.g., further to the left of receptacle 121 as shown in the view of FIG. 5). Enlarging buttress 123 in this manner allows for an even greater mass and thereby weight of in-process build structure to overlie build plate 150 without overlying any portion of support structure 120 when viewed in a downward direction orthogonal to build plate 140.

Each of in-process build structure 110, support structure 120, and the powder particles of in-process build assembly 100 may be formed in a layer-by-layer fashion during an AM process using a high energy beam, which may be but is not limited to being an SLS, SLM, DMLS, or EBM process, e.g., the processes described in U.S. Patent Nos. 7,537,664; 8,728,387; 9,456,901; and 10,614,176, the disclosures of each of which being hereby incorporated by reference herein. Indeed, fabrication of the entirety of in-process build assembly 100 into the form shown in FIGS. 1 and 5 may be performed during a single AM process. The AM process may use a compatible computer file, such as an . STL file, corresponding to a CAD model of the in-process build assembly 100 or components, e.g., in-process build structure 110 and support structure 120, of the in-process build assembly.

In forming in-process build assembly 100, a first layer or portion of a layer of metal powder is deposited onto a substrate, such as build plate 140, and then a high energy beam is applied to the metal powder, e.g., by scanning the high energy beam, so as to sinter or melt the powder and create solid portions or open portions, which may be in the form of any one or any combination of holes, windows 127, perforations significantly smaller than the windows, or porous geometries. Successive layers of the metal powder are then deposited onto previous layers of the metal powder, and the high energy beam is applied respectively to each of the successive layers prior to the deposition of subsequent layers of the metal powder. The scanning and depositing of successive layers of the metal powder continues the building process until the entirety of in-process build assembly 100 is formed. Such continuation of the building process forms portions of only support structure 120 within a first set of formed metal layers, portions of each of the support structure, in-process build structure 110, and the powder particles within a second set of formed metal layers, portions of only a combination of the support structure and the in-process build structure within a third set of formed metal layers, and portions of only the in-process build structure within a fourth set of formed metal layers.

The structures formed using this process may be partially porous and, if desired, have interconnecting pores to provide an interconnecting porosity. In fabricating in-process build system 150, support structure 120 of in-process build assembly 100 may be fixed to build plate 140 during the entirety of the fabrication of the in-process build assembly, e.g., a femoral hip stem. Any one or any combination of in-process build structure 110, support structure 120, and the powder particles preferably may be made of any of cobalt chrome alloy, titanium or alloy, stainless steel, niobium, tantalum, a ceramic material, a polymeric or other plastic material, or any combination thereof. In this manner, in-process build assembly 100 may be fabricated with a single material or a mixture of materials. In alternative arrangements, in-process build assembly 100 could be formed of other materials, including plastics (e.g., via fused deposition modeling (FDM), fused filament fabrication (FFF), digital light processing (DLP), plastic powder sintering), ceramics (e.g., via extrusion, ceramic powder sintering, (DLP)), or biomaterials (e.g., via SLS and FDM) using appropriate 3D printing machines known to those skilled in the art.

The high energy beam preferably may be an electron beam (e-beam) or laser beam and may be applied continuously to the powder or pulsed at a predetermined frequency. In some arrangements, the use of a laser or e-beam melting process may preclude the requirement for subsequent heat treatment of the structure fabricated by the additive manufacturing process, e.g., of in-process build assembly 100 or of only in-process build structure 110. The high energy beam is emitted from a beam-generating apparatus to heat the metal powder sufficiently to sinter and preferably to at least partially melt or fully melt the metal powder, or in the case of the semi-sintered powder particles, to heat the metal powder sufficiently to attach the particles to both in-process build structure 110 and support structure 120 without sintering the powder particles.

High energy beam generation equipment for manufacturing such structures may be one of many currently available including the "Concept laser M2 Cusing" machines, GE Concept M2 Cusing Gen 2 machines, GE Arcam Q10 machines, 200W M2 Cusing (series 3), kW M2 Cusing (Series 3), Dual kW M2 Cusing (Series 5) MCP REALIZER, the EOS M270, TRUMPF TRUMAFORM 250, the ARCAM EBM S12 and Q10 machine, and the like. These machines are listed by way of example only. Thus, this list suggests which machines may have suitable capabilities for the processes described herein, but any other machines having similar capabilities may be used instead. The beam generation equipment may also be a custom-produced laboratory device.

The pore density, pore size and pore size distribution may be controlled from one location on the structure to another. It is important to note that successive powder layers may differ in porosity by varying factors used for laser scanning powder layers. Additionally, the porosity of successive layers of powder may be varied by either creating a specific type of unit cell or manipulating various dimensions of a given unit cell. In some arrangements, the porosity may be a gradient porosity throughout at least a portion of the fabricated structure. The use of any of the holes, windows 127, lower central bore 124, perforations, and porous geometries is useful to reduce the time for the AM process to fabricate in-process build assembly 100.

The beam generation equipment may be programmed to proceed in a random generated manner to produce an irregular porous construct but with a defined level of porosity. Pseudo-random geometries may be formed by applying a perturbation to the vertices of porous geometries when preparing model build structures corresponding to the 3D structure to be fabricated. In this manner, the porous geometries may be randomized.

Support structure 120 could be utilized with any in-process build structure utilizing trunnion 115 or other structure of same dimensions as the trunnion. As such, CAD models and related AM component files for use in fabricating support structure 120 in an additive manufacturing process such as those described above as well as CAD models and related AM component files for use in fabricating trunnion 115 in the additive manufacturing process could be utilized with CAD models and related AM component files for necks and stems different from neck 112 and stem 114.

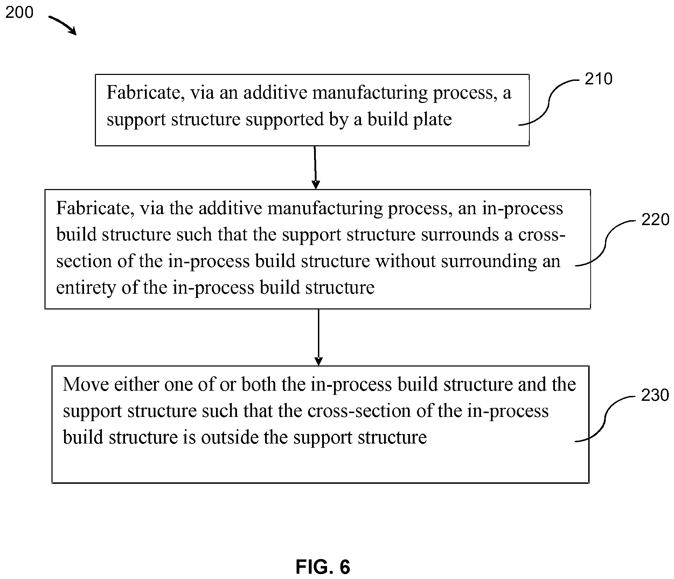

Referring now to FIG. 6, a build structure, e.g., in-process build assembly 100, is fabricated, at least in part, by process 200. At step 210 of this process, a support structure, e.g., support structure 120, is fabricated, via an additive manufacturing process, by a build plate. At step 220 of this process, an in-process build structure, e.g., in-process build structure 110, is fabricated, via the additive manufacturing process, such that the support structure surrounds a cross-section of the in-process build structure. At step 230 of this process, either one of or both the in-process build structure and the support structure are moved, e.g., by removing trunnion 115 of in-process build structure 110 from receptacle 121 of support structure 120, such that the cross-section of the in-process build structure is outside the support structure.

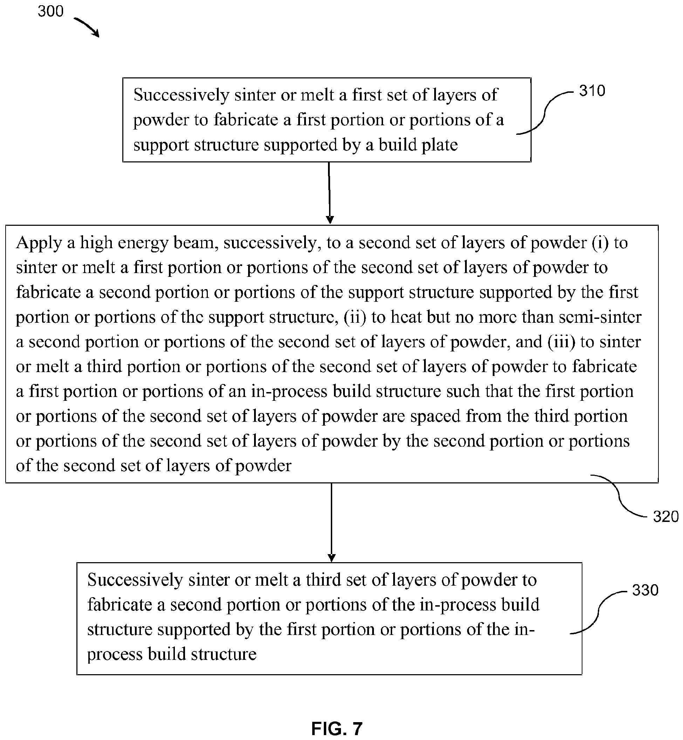

With reference now to FIG. 7, a build structure, e.g., in-process build assembly 100, is fabricated, at least an in part, by process 300. At step 310 of this process, first layers of powder, e.g., metal powder, are successively sintered or melted to fabricate a first portion or portions of a support structure, e.g., support structure 120, supported by a build plate, e.g., build plate 140. At step 320 of this process, a high energy beam is applied, e.g., by an additive layer manufacturing machine such as those discussed above, to second layers of powder which may be metal powder, successively, (i) to sinter or melt a first portion or portions of second layers of powder to fabricate a second portion or portions of the support structure supported by the first portion or portions of the support structure, (ii) to no more than semi-sinter a second portion or portions of the second layers of powder, and (iii) to sinter or melt a third portion or portions of the second layers of powder to fabricate a first portion or portions of an in-process build structure, e.g., in-process build structure 110, such that the first portion or portions of the second layers of powder are spaced from the third portion or portions of the second layers of powder by the second portion or portions of the second layers of powder. By way of step 320, the second portion or portions of the second layers powder are between the first portions of powder and the third portions of powder. At step 330 of this process, third layers of powder, e.g., metal powder, are successively sintered or melted to fabricate a second portion or portions of the in-process build structure.

It is to be understood that the disclosure set forth herein includes any possible combinations of the particular features set forth above, whether specifically disclosed herein or not. For example, where a particular feature is disclosed in the context of a particular aspect, arrangement, configuration, or embodiment, that feature can also be used, to the extent possible, in combination with and/or in the context of other particular aspects, arrangements, configurations, and embodiments of the invention, and in the invention generally.

Furthermore, although the invention disclosed herein has been described with reference to particular features, it is to be understood that these features are merely illustrative of the principles and applications of the present invention. It is therefore to be understood that numerous modifications may be made to the illustrative embodiments and that other arrangements may be devised without departing from the spirit and scope of the present invention. In this regard, the present invention encompasses numerous additional features in addition to those specific features set forth in the claims below.

## Claims

1. A method of fabricating a build structure, comprising steps of:
fabricating, via an additive manufacturing process, a support structure (120) supported by a build plate (140);
fabricating, via the additive manufacturing process, an in-process build structure (110) such that the support structure surrounds a cross-section of the in-process build structure without surrounding an entirety of the in-process build structure; and
moving either one of or both the in-process build structure and the support structure such that the cross-section of the in-process build structure is outside the support structure.

2. The method of claim 1, further comprising a step of attaching the in-process build structure to the support structure with semi-sintered particles.

3. The method of claim 1 or claim 2, wherein the fabricating steps form the in-process build structure and the support structure such that a first portion of the in-process build structure that includes the cross-section of the in-process build structure is held within the support structure.

4. The method of claim 3 when dependent on claim 2, wherein the fabricating steps include applying a high energy beam onto a powder layer to form the first portion of the in-process build structure, a first portion of the support structure, and semi-sintered particles between the in-process build structure and the support structure.

5. The method of any one of claims 1-4, further comprising a step of altering a first surface of the in-process build structure when the in-process build structure is outside the support structure.

6. The method of claim 5, wherein the altering step includes lowering a surface roughness of a first region of the first surface of the in-process build structure.

7. The method of claim 5 or claim 6, wherein the altering step includes sandblasting the support structure attached to the in-process build structure.

8. The method of any one of claims 1-7, further comprising either one of or both twisting and pulling the in-process build structure relative to the support structure such that the in-process build structure detaches from the support structure.

9. The method of any one of claims 1-8, wherein the in-process build structure fabricating step fabricates the in-process build structure in the form of a femoral hip stem.

10. The method of claim 9, wherein the in-process build structure fabricating step fabricates the in-process build structure such that the support structure surrounds a portion of the in-process build structure in the form of a trunnion head (115) of a femoral hip stem.

11. The method of any one of claims 1-10, wherein both the support structure and the in-process build structure are made of metal.

12. An in-process build assembly (100), comprising:
an in-process build structure (110);
a support structure (120) supporting the in-process build structure and surrounding a cross-section of the in-process build structure without surrounding an entirety of the in-process build structure; and
semi-sintered particles located between the in-process build structure and the support structure such that there is no direct attachment of the in-process build structure to the support structure.

13. The in-process build assembly of claim 12,
wherein the support structure comprises a receptacle (121) and a buttress (123) extending from the receptacle, and
wherein the receptacle surrounds the cross-section of the in-process build structure.

14. The in-process build assembly claim 13, wherein the semi-sintered particles are located in and supported by the receptacle.

15. An in-process build system (150), comprising:
the in-process build assembly of claim 13 or claim 14; and
a build plate (140) attached to the receptacle of the support structure such that the build plate fully supports the in-process build assembly.
